(19) Europäisches Patentamt
European Patent Office
Office européen des brevets

(11) **EP 3 899 968 B1**

(12) **FASCICULE DE BREVET EUROPEEN**

(45) Date de publication et mention
de la délivrance du brevet:
**20.11.2024 Bulletin 2024/47**

(21) Numéro de dépôt: **19848804.1**

(22) Date de dépôt: **20.12.2019**

(51) Classification Internationale des Brevets (IPC):
**G16H 20/17** $^{(2018.01)}$

(52) Classification Coopérative des Brevets (CPC):
**G16H 20/17**

(86) Numéro de dépôt international:
**PCT/FR2019/053237**

(87) Numéro de publication internationale:
**WO 2020/128387 (25.06.2020 Gazette 2020/26)**

(54) **SYSTÈME AUTOMATISÉ DE RÉGULATION DE LA GLYCÉMIE D'UN PATIENT**

AUTOMATISIERTES SYSTEM ZUR REGULIERUNG DES BLUTZUCKERSPIEGELS EINES PATIENTEN

AUTOMATED SYSTEM FOR REGULATING THE BLOOD GLUCOSE LEVEL OF A PATIENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.12.2018 FR 1873812**

(43) Date de publication de la demande:
**27.10.2021 Bulletin 2021/43**

(73) Titulaire: **Commissariat à l'Energie Atomique et aux Energies Alternatives**
**75015 Paris (FR)**

(72) Inventeurs:
• **BLANC, Romain**
**38000 GRENOBLE (FR)**
• **DORON, Eléonore-Maeva**
**38054 GRENOBLE CEDEX 9 (FR)**
• **VILLENEUVE, Emma**
**38054 GRENOBLE CEDEX 9 (FR)**

(74) Mandataire: **Cabinet Beaumont**
**4, Place Robert Schuman**
**B.P. 1529**
**38025 Grenoble Cedex 1 (FR)**

(56) Documents cités:
**GB-A- 2 436 873    US-A1- 2014 118 138**

• **SARA LAJEUNESSE: "Software program can predict blood sugar crashes, spikes for patients with type 1 diabetes", 26 February 2014 (2014-02-26), XP055615579, Retrieved from the Internet <URL:https://medicalxpress.com/news/2014-02-software-blood-sugar-spikes-patients.html> [retrieved on 20190826]**
• **ROMAN HOVORKA ET AL: "Nonlinear model predictive control of glucose concentration in subjects with type 1 diabetes", PHYSIOLOGICAL MEASUREMENT., vol. 25, no. 4, 23 July 2004 (2004-07-23), GB, pages 905 - 920, XP055420209, ISSN: 0967-3334, DOI: 10.1088/0967-3334/25/4/010**
• **ROMAN HOVORKA ET AL: "Partitioning glucose distribution/transport, disposal, and endogenous production during IVGTT", AMERICAN JOURNAL OF PHYSIOLOGY: ENDOCRINOLOGY AND METABOLISM., vol. 282, no. 5, 1 May 2002 (2002-05-01), US, pages E992 - E1007, XP055420211, ISSN: 0193-1849, DOI: 10.1152/ajpendo.00304.2001**

EP 3 899 968 B1

**Description**

Domaine technique

La présente demande concerne le domaine des systèmes automatisés de régulation de glycémie, aussi appelés pancréas artificiels.

Technique antérieure

Un pancréas artificiel est un système permettant de réguler automatiquement les apports en insuline d'un patient diabétique à partir de son historique de glycémie (ou taux de glucose sanguin), de son historique de prise de repas, et de son historique d'injection d'insuline.

Des exemples de systèmes de régulation de ce type sont notamment décrits dans les demandes de brevet WO2018/055283 (DD16959/B15018) et WO2018/055284 (DD17175/B15267) précédemment déposées par le demandeur.

Le document US 2014/118138 A1, COBELLI et al, 1 mai 2014, divulgue un système d'alerte par rapport à la condition glycémique qui, selon le cas, commande l'interruption du débit d'insuline injectée au patient ou déclenche une alerte de resucrage.

Il serait souhaitable de pouvoir améliorer les performances des pancréas artificiels connus, et notamment de pouvoir limiter davantage les risques de placer le patient en situation d'hyperglycémie ou d'hypoglycémie.

Résumé de l'invention

L'invention est définie par les revendications annexées.

Ainsi, l'invention prévoit un système automatisé de régulation de la glycémie d'un patient, comportant :

- un capteur de glycémie ;
- un dispositif d'injection d'insuline ; et
- une unité de traitement et de contrôle,

dans lequel l'unité de traitement et de contrôle est configurée pour mettre en oeuvre un procédé de minimisation d'hypoglycémie comprenant les étapes suivantes :

a) détecter une possible hypoglycémie à venir à partir de mesures de glycémie fournies par le capteur de glycémie ;
b) déterminer une valeur BGdebt représentative d'une dette de glycémie à compenser pour éviter l'hypoglycémie détectée à l'étape a) et une valeur Thypo représentative de la durée restante avant le début de l'hypoglycémie détectée à l'étape a) ;
c) déterminer une valeur Tautosugaring représentative de la durée nécessaire pour permettre au patient de compenser la dette de glycémie BGdebt déterminée à l'étape b) par production endogène de glucose ;
d) comparer la valeur Tautosugaring déterminée à l'étape c) à une valeur Thypo-TH, où TH est une marge temporelle prédéterminée ; et
e) commander l'interruption du débit d'insuline injecté au patient par le dispositif d'injection d'insuline et/ou déclencher une alerte via un dispositif d'interface utilisateur du système pour indiquer au patient qu'il doit procéder à un resucrage lorsqu'il est déterminé à l'étape d) que la valeur Tautosugaring est supérieure ou égale à la valeur Thypo-TH.

Dans l'invention, à l'étape c), l'unité de traitement et de contrôle détermine une valeur EGE représentative de la vitesse de remontée glycémique endogène du patient, la valeur Tautosugaring étant ensuite calculée par l'unité de traitement et de contrôle selon la formule suivante :

[Math 1]

$$\text{Tautosugaring} = \frac{\text{BGdebt}}{\text{EGE}}$$

Selon un mode de réalisation, à l'étape c), la valeur EGE est calculée par l'unité de traitement et de contrôle par analyse de l'historique de glycémie et de l'historique d'injection d'insuline du patient.

Selon un mode de réalisation, à l'étape e), l'unité de traitement et de contrôle détermine une valeur predBGΔThypo estimative de la glycémie future du patient à l'issue de la période Thypo.

Selon un mode de réalisation, l'unité de traitement et de contrôle est configurée pour, après l'étape e), mettre en oeuvre les étapes suivantes :

> f) comparer la valeur predBG∆Thypo à un seuil d'hypoglycémie BGlimhypo prédéterminé ;
> g) lorsqu'il est déterminé à l'étape f) que la valeur predBG∆Thypo est inférieure au seuil d'hypoglycémie BGlimhypo, comparer une valeur BG(t0) représentative de la glycémie courante du patient, mesurée par le capteur, à une valeur BGlimhypo+T1, où T1 est une marge de glycémie prédéterminée ; et
> h) lorsqu'il est déterminé à l'étape g) que la valeur BG(t0) est inférieure à la valeur BGlimhypo+T1, déclencher une alerte via un dispositif d'interface utilisateur du système pour indiquer au patient qu'il doit procéder à un resucrage.

Selon un mode de réalisation, à l'étape b), l'unité de traitement et de contrôle détermine trois valeurs Thypolim, Thypomean et Thypocurrent estimatives de la durée restante avant le début de l'hypoglycémie, basées respectivement sur un taux de chute de glycémie maximal nGRClim déterminé pour le patient, sur un taux de chute de glycémie moyen nGRCmean estimé pour le patient, et sur un taux de chute de glycémie courant nGRCcurrent estimé pour le patient.

Selon un mode de réalisation, l'unité de traitement et de contrôle est configurée pour, à l'étape d), comparer la durée Tautosugaring à une valeur ThypoMIN-TH, où ThypoMIN est la plus courte des trois durées Thypolim, Thypomean et Thypocurrent.

Selon un mode de réalisation, l'unité de traitement et de contrôle est configurée pour, à l'étape e), calculer trois valeurs predBG∆Thypolim, predBG∆Thypomean et predBG∆Thypocurrent estimatives de la glycémie future du patient respectivement à l'issue de la période Thypolim, à l'issue de la période Thypomean et à l'issue de la période Thypocurrent.

Selon un mode de réalisation, l'unité de traitement et de contrôle est configurée pour, après l'étape e), mettre en oeuvre les étapes suivantes :

> f') comparer chacune des trois valeurs predBG∆Thypolim, predBG∆Thypomean et predBG∆Thypocurrent à un seuil d'hypoglycémie BGlimhypo prédéterminé ; et
> g') si une seule des trois valeurs predBG∆Thypolim, predBG∆Thypomean et predBG∆Thypocurrent est inférieure au seuil BGlimhypo, proposer un resucrage au patient uniquement si une valeur BG(t0) représentative de la glycémie courante du patient est inférieure à une valeur BGlimhypo+T1, où T1 est une marge de glycémie prédéterminée, si deux des trois valeurs predBG∆Thypolim, predBG∆Thypomean et predBG∆Thypocurrent sont inférieures au seuil BGlimhypo, proposer un resucrage au patient uniquement si la valeur BG(t0) est inférieure à une valeur BGlimhypo+T2, où T2 est une marge de glycémie prédéterminée supérieure à la marge T1, et si les trois valeurs predBG∆Thypolim, predBG∆Thypomean et predBG∆Thypocurrent sont inférieures au seuil BGlimhypo, proposer un resucrage au patient uniquement si la valeur BG(t0) est inférieure à une valeur BGlimhypo+T3, où T3 est une marge de glycémie prédéterminée supérieure à la marge T2.

Selon un mode de réalisation, le système comprend en outre un dispositif de mesure d'une activité physique du patient relié à l'unité de traitement et de contrôle et communiquant à l'unité de traitement et de contrôle un signal iap(t) représentatif de l'évolution, en fonction du temps, d'une activité physique du patient.

Selon un mode de réalisation, l'unité de traitement et de contrôle tient compte du signal iap(t) pour détecter une possible hypoglycémie à venir à l'étape a) et/ou pour déterminer la valeur Tautosugaring à l'étape c).

Selon un mode de réalisation, à l'étape c), l'unité de traitement et de contrôle détermine un signal IOB(t) représentatif de la quantité d'insuline embarquée du patient, défini par multiplication d'un signal représentatif de la quantité d'insuline injectée au patient par une exponentielle décroissante de constante de temps $\tau$IOBap, la constante de temps $\tau$IOBap étant une fonction décroissante du signal iap(t), modélisant l'augmentation de la vitesse d'action de l'insuline lorsque l'intensité de l'activité physique augmente.

Selon un mode de réalisation, la constante de temps $\tau$IOBap est définie comme suit :

[Math 2]

$$\tau IOBap = \frac{C}{iap(t)}$$

où C est un paramètre compris entre 1 et 100.

Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :

la figure 1 représente de façon schématique, sous forme de blocs, un exemple d'un système automatisé de régulation de la glycémie d'un patient selon un mode de réalisation ;

la figure 2 est une représentation simplifiée d'un modèle physiologique pouvant être utilisé dans le système de la figure 1 pour prédire l'évolution future de la glycémie du patient ;

la figure 3 est un diagramme illustrant un exemple d'un procédé automatisé de régulation de glycémie pouvant être mis en oeuvre par le système de la figure 1 ;

la figure 4 est un diagramme illustrant un exemple d'un mode de réalisation d'un procédé automatisé de régulation de glycémie mis en oeuvre par le système de la figure 1 ;

la figure 5 est un diagramme illustrant plus en détail un exemple de mise en oeuvre du procédé de régulation de la figure 4 ;

la figure 6 est un diagramme illustrant un exemple de procédé de détermination d'une valeur représentative de la production endogène de glucose d'un patient ;

la figure 7 est un autre diagramme illustrant un exemple de procédé de détermination d'une valeur représentative de la production endogène de glucose d'un patient ; et

la figure 8 représente de façon schématique, sous forme de blocs, un autre exemple d'un système automatisé de régulation de la glycémie d'un patient selon un mode de réalisation.

Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Par souci de clarté, seuls les étapes et éléments utiles à la compréhension des modes de réalisation décrits ont été représentés et sont détaillés. En particulier, les dispositifs de mesure de glycémie et les dispositifs d'injection d'insuline des systèmes de régulation décrits n'ont pas été détaillés, les modes de réalisation décrits étant compatibles avec tous ou la plupart des dispositifs de mesure de glycémie et d'injection d'insuline connus. De plus, la réalisation matérielle de l'unité de traitement et de contrôle des systèmes de régulation décrits n'a pas été détaillée, la réalisation d'une telle unité de traitement et de contrôle étant à la portée de l'homme du métier à partir des indications fonctionnelles de la présente description.

Sauf précision contraire, les expressions "environ", "approximativement", "sensiblement", et "de l'ordre de" signifient à 10 % près, de préférence à 5 % près.

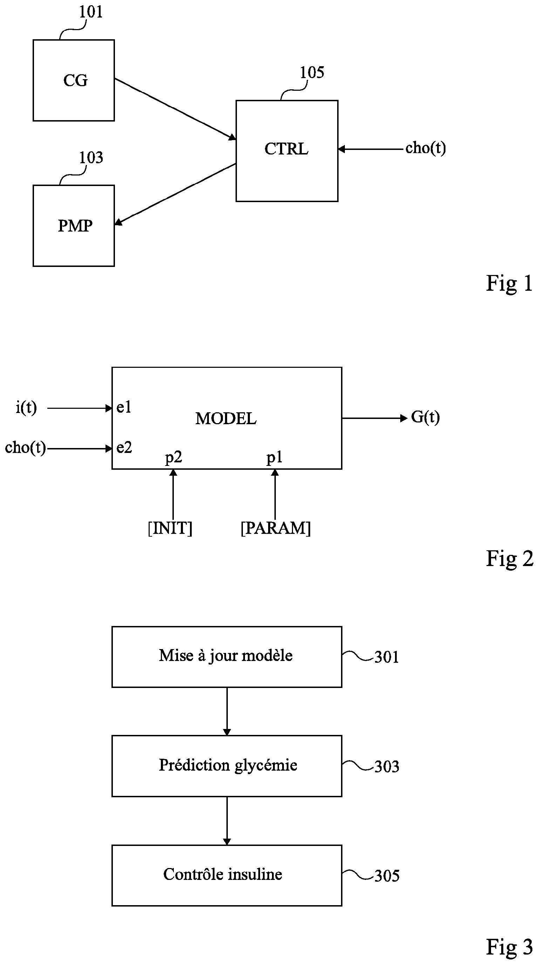

La figure 1 représente de façon schématique, sous forme de blocs, un exemple d'un mode de réalisation d'un système automatisé de régulation de la glycémie d'un patient.

Le système de la figure 1 comprend un capteur 101 (CG) adapté à mesurer la glycémie du patient. En fonctionnement normal, le capteur 101 peut être positionné à demeure sur ou dans le corps du patient, par exemple à hauteur de son abdomen. Le capteur 101 est par exemple un capteur de type CGM (de l'anglais "Continuous Glucose Monitoring" - surveillance continue de glycémie), c'est-à-dire un capteur adapté à mesurer en continu ou à une fréquence relativement élevée (par exemple au moins une fois toutes les vingt minutes et de préférence au moins une fois toutes les cinq minutes) la glycémie du patient. Le capteur 101 est par exemple un capteur de glycémie en sous-cutané.

Le système de la figure 1 comprend en outre un dispositif d'injection d'insuline 103 (PMP), par exemple un dispositif d'injection en sous-cutané. Le dispositif 103 est par exemple un dispositif d'injection automatique de type pompe à insuline, comportant un réservoir d'insuline relié à une aiguille d'injection implantée sous la peau du patient, la pompe pouvant être commandée électriquement pour injecter automatiquement des doses d'insuline déterminées à des instants déterminés. En fonctionnement normal, le dispositif d'injection 103 peut être positionné à demeure dans ou sur le corps du patient, par exemple au niveau de son abdomen.

Le système de la figure 1 comprend en outre une unité de traitement et de contrôle 105 (CTRL) reliée d'une part au capteur de glycémie 101, par exemple par liaison filaire ou par liaison radio (sans fil), et d'autre part au dispositif d'injection 103, par exemple par liaison filaire ou radio. En fonctionnement, l'unité de traitement et de contrôle 105 est adaptée à recevoir les données de glycémie du patient mesurées par le capteur 101, et à commander électriquement le dispositif 103 pour injecter au patient des doses d'insuline déterminées à des instants déterminés. Dans cet exemple, l'unité de traitement et de contrôle 105 est en outre adaptée à recevoir, par l'intermédiaire d'une interface utilisateur non détaillée, des données cho(t) représentatives de l'évolution, en fonction du temps, de la quantité de glucose ingérée par le patient.

L'unité de traitement et de contrôle 105 est adaptée à déterminer les doses d'insuline à injecter au patient en tenant compte notamment de l'historique de glycémie mesurée par le capteur 101, de l'historique d'insuline injectée par le dispositif 103, et de l'historique d'ingestion de glucose par le patient. Pour cela, l'unité de traitement et de contrôle 105 comprend un circuit de calcul numérique (non détaillé), comprenant par exemple un microprocesseur. L'unité de traitement et de contrôle 105 est par exemple un dispositif mobile transporté par le patient tout au long de la journée et/ou de la nuit, par exemple un dispositif de type smartphone configuré pour mettre en oeuvre un procédé de régulation du type décrit ci-après.

L'unité de traitement et de contrôle 105 est par exemple configurée pour mettre en oeuvre un procédé automatisé de régulation de type MPC (de l'anglais "Model-based Predictive Control"), aussi appelé procédé de régulation à commande prédictive, dans lequel la régulation de la dose d'insuline administrée tient compte d'une prédiction de l'évolution future de la glycémie du patient en fonction du temps réalisée à partir d'un modèle mathématique, par exemple un modèle physiologique décrivant l'assimilation de l'insuline par le corps du patient et son impact sur la glycémie du patient.

Plus particulièrement, l'unité de traitement et de contrôle 105 peut être configurée pour, à partir de l'historique d'insuline injectée et de l'historique de glucose ingéré, et en se basant sur un modèle mathématique prédéterminé, déterminer une courbe représentative de l'évolution attendue de la glycémie du patient en fonction du temps, sur une période à venir appelée période de prédiction ou horizon de prédiction, par exemple une période de 1 à 10 heures. En tenant compte de cette courbe, l'unité de traitement et de contrôle 105 détermine les doses d'insuline qu'il conviendrait d'injecter au patient pendant la période de prédiction à venir, pour que la glycémie réelle (par opposition à la glycémie estimée à partir du modèle) du patient reste dans des limites acceptables, et en particulier pour limiter les risques d'hyperglycémie ou d'hypoglycémie.

Dans ce mode de fonctionnement, comme cela sera expliqué plus en détail ci-après, les données de glycémie réelle mesurées par le capteur 101 sont utilisées principalement à des fins de calibration du modèle mathématique.

La figure 2 est une représentation simplifiée d'un modèle mathématique MODEL utilisé dans le système de la figure 1 pour prédire l'évolution future de la glycémie du patient. Sur la figure 2, le modèle est représenté sous la forme d'un bloc de traitement comportant :

une entrée e1 sur laquelle est appliqué un signal i(t) représentatif de l'évolution, en fonction du temps t, de la quantité d'insuline injectée au patient ;

une entrée e2 sur laquelle est appliquée un signal cho(t) représentatif de l'évolution, en fonction du temps t, de la quantité de glucose ingérée par le patient ; et

une sortie s fournissant un signal G(t) représentatif de l'évolution, en fonction du temps t, de la glycémie du patient.

Le modèle MODEL peut être un modèle physiologique, par exemple un modèle compartimental comportant, outre les variables d'entrée i(t) et cho(t) et la variable de sortie G(t), une pluralité de variables d'états correspondant à des variables physiologiques du patient, évoluant en fonction du temps. L'évolution temporelle des variables d'état et de la variable de sortie G(t) est régie par un système d'équations différentielles comportant une pluralité de paramètres représentés sur la figure 2 par un vecteur [PARAM] appliqué sur une entrée p1 du bloc MODEL. La réponse du modèle physiologique est en outre conditionnée par les états initiaux ou valeurs initiales affectés aux variables d'état, représentés sur la figure 2 par un vecteur [INIT] appliqué sur une entrée p2 du bloc MODEL.

A titre d'exemple, le modèle MODEL est le modèle physiologique dit de Hovorka, décrit dans l'article intitulé "Nonlinear model prédictive control of glucose concentration in subjects with type 1 diabètes" de Roman Hovorka et al. (Physiol Meas. 2004;25:905-920), et dans l'article intitulé "Partitioning glucose distribution/transport, disposal, and endogenous production during IVGTT", de Roman Hovorka et al. (Am J Physiol Endocrinol Metab 282: E992-E1007, 2002). Plus généralement, tout autre modèle physiologique décrivant l'assimilation de l'insuline par le corps d'un patient et son effet sur la glycémie du patient peut être utilisé, par exemple le modèle dit de Cobelli, décrit dans l'article intitulé "A System Model of Oral Glucose Absorption: Validation on Gold Standard Data", de Chiara Dalla Man et al. (IEEE TRANSACTIONS ON BIOMEDICAL ENGINEERING, VOL. 53, NO. 12, DECEMBER 2006) .

Parmi les paramètres du vecteur [PARAM], certains peuvent être considérés comme constants pour un patient donné. D'autres paramètres, appelés ci-après paramètres temps-dépendants, sont en revanche susceptibles d'évoluer dans le temps. Du fait de cette variabilité de certains paramètres du système, on peut prévoir de ré-étalonner ou re-calibrer régulièrement le modèle en cours d'utilisation, par exemple à chaque acquisition d'une nouvelle valeur de glycémie par le capteur 101, par exemple toutes les 1 à 20 minutes, par exemple toutes les 5 minutes, pour s'assurer que les prédictions du modèle restent pertinentes. Cette mise à jour du modèle, aussi appelée personnalisation du modèle, est réalisée de façon automatique par le système de la figure 1, c'est-à-dire sans qu'il soit nécessaire de mesurer physiquement les paramètres temps-dépendants du système sur le patient puis de les transmettre à l'unité de traitement et de contrôle 105.

La figure 3 est un diagramme illustrant un exemple d'un procédé automatisé de régulation de glycémie pouvant être mis en oeuvre par le système de la figure 1.

Ce procédé comprend une étape 301 de re-calibration ou mise à jour du modèle. Lors de cette étape, l'unité de traitement et de contrôle 105 met en oeuvre un procédé de ré-estimation des paramètres temps-dépendants du modèle en tenant compte des données d'insuline effectivement injectée par le dispositif 103 et des données de glycémie réelle mesurée par le capteur 101 pendant une période d'observation passée de durée $\Delta T$, par exemple une période de 1 à 10 heures précédant l'étape de calibration. Plus particulièrement, lors de l'étape de calibration, l'unité de traitement et de contrôle 105 simule le comportement du patient sur la période d'observation passée à partir du modèle physiologique (en tenant compte des éventuelles ingestions de glucose et injections d'insuline pendant cette période), et compare la courbe de glycémie estimée par le modèle à la courbe de glycémie réelle mesurée par le capteur pendant cette même période. L'unité de traitement et de contrôle 105 recherche alors, pour les paramètres temps-dépendants du modèle, un jeu de valeurs conduisant à minimiser une grandeur représentative de l'erreur entre la courbe de glycémie estimée par le modèle et la courbe de glycémie réelle pendant la période d'observation. A titre d'exemple, l'unité de traitement et de contrôle recherche un jeu de paramètres conduisant à minimiser un indicateur m représentatif de l'aire entre la courbe de glycémie estimée par le modèle et la courbe de glycémie réelle pendant la période d'observation, aussi appelé écart quadratique moyen entre la glycémie estimée et la glycémie réelle, par exemple défini comme suit :

[Math 3]

$$m = \frac{1}{\Delta T} \sum_{t=t_0-\Delta T}^{t_0} |g(t) - gh(t)|^2$$

où t est la variable temps discrétisée, $t_0-\Delta T$ correspond à l'instant de début de la phase d'observation passé, $t_0$ correspond à l'instant de fin de la phase d'observation passée (correspondant par exemple à l'instant de début de l'étape de calibration du modèle), g est la courbe d'évolution temporelle de la glycémie réelle mesurée par le capteur 101 pendant la période $[t_0-\Delta T, t_0]$, et gh est la courbe de glycémie estimée à partir du modèle pendant la période $[t_0-\Delta T, t_0]$. A titre de variante, pour le calcul de l'écart quadratique moyen, la variable $\Delta T$ peut être remplacée par le nombre de mesures réalisées pendant la période d'observation passée. L'algorithme de recherche de paramètres optimaux utilisé lors de cette étape n'est pas détaillé dans la présente demande, les modes de réalisation décrits étant compatibles avec les algorithmes usuels utilisés dans des domaines variés pour résoudre des problèmes d'optimisation de paramètres par minimisation d'une fonction de coût.

On notera que lors de l'étape 301, outre les paramètres temps-dépendants du modèle, l'unité de traitement et de contrôle 105 définit un vecteur [INIT] d'états initiaux (états à l'instant $t_0-\Delta T$) des variables d'état du modèle, pour pouvoir simuler le comportement du patient à partir du modèle. Pour définir les états initiaux des variables d'état du modèle, une première possibilité consiste à faire l'hypothèse que, dans la période précédant la période d'observation $[t_0-\Delta T, t_0]$ sur laquelle est basée la calibration du modèle, le patient se trouvait dans un état stationnaire, avec un débit d'insuline injectée constant, et une prise alimentaire de glucose nulle. Sous cette hypothèse, toutes les dérivées du système d'équations différentielles peuvent être considérées comme nulles à l'instant initial $t_0-\Delta T$. Les valeurs à l'instant $t_0-\Delta T$ des variables d'état du système peuvent alors être calculées analytiquement. Pour améliorer l'initialisation, une autre possibilité consiste à faire les mêmes hypothèses que précédemment, mais en ajoutant la contrainte que la glycémie estimée à l'instant $t_0-\Delta T$ soit égale à la glycémie réelle mesurée par le capteur. Pour améliorer encore l'initialisation, une autre possibilité est de considérer les états initiaux des variables d'état du modèle comme des variables aléatoires, au même titre que les paramètres temps-dépendants du modèle. Les états initiaux des variables d'état sont alors déterminés de la même façon que les paramètres temps-dépendants du modèle, c'est-à-dire que l'unité de traitement et de contrôle 105 recherche un jeu de valeurs d'états initiaux [INIT] conduisant à minimiser une grandeur représentative de l'erreur entre la courbe de glycémie estimée par le modèle et la courbe de glycémie réelle pendant la période d'observation passée.

Le procédé de la figure 3 comprend en outre, après l'étape 301, une étape 303 de prédiction, par l'unité de

6

traitement et de contrôle 105, de l'évolution temporelle de la glycémie du patient sur une période de prédiction à venir [t0, t0+Tpred] de durée Tpred, par exemple comprise entre 1 et 10 heures, à partir du modèle physiologique mis à jour à l'étape 301 et en tenant compte de l'historique d'insuline injectée au patient et de l'historique de glucose ingéré par le patient.

Le procédé de la figure 3 comprend de plus, après l'étape 303, une étape 305 de détermination, par l'unité de traitement et de contrôle 105, en tenant compte de la courbe de glycémie future prédite à l'étape 303, des doses d'insuline à injecter au patient pendant la période de prédiction à venir [t0, t0+Tpred]. A l'issue de cette étape, l'unité de traitement et de contrôle 105 peut programmer le dispositif d'injection 103 pour administrer les doses déterminées pendant la période de prédiction [t0, t0+Tpred].

La suite d'étapes 301, 303 et 305 peut être répétée à intervalles réguliers, par exemple à chaque nouvelle acquisition d'une valeur de glycémie par le capteur 101, par exemple toutes les 1 à 20 minutes, par exemple toutes les cinq minutes.

A titre de variante, le modèle MODEL peut être un modèle mathématique non physiologique, par exemple un modèle autorégressif de type ARX, sous la forme d'une ou plusieurs équations déterminées par apprentissage par la seule observation des effets des variables d'entrée sur la sortie G(t), sans tenir compte des différents mécanismes physiologiques connus s'opérant dans le corps du patient

Dans le système décrit ci-dessus, pour garantir la sécurité du patient, la commande du dispositif d'injection d'insuline n'est pas effectuée sur la seule base des données de sortie du modèle MODEL. Plus particulièrement, le modèle MODEL est utilisé en combinaison avec un algorithme chapeau de sécurité, aussi appelé hypominimizer (HM), ou algorithme de minimisation d'hypoglycémie, ayant pour fonction d'anticiper et de prévenir les hypoglycémies imminentes en interrompant le débit d'insuline administré par le dispositif 103 et/ou en proposant au patient un resucrage, c'est-à-dire une ingestion de glucose.

En effet, dans certaines situations, il peut arriver que les prédictions réalisées par le modèle mathématique MODEL ne soient pas suffisamment fiables, et que par conséquent la commande du dispositif d'injection d'insuline 103 sur la seule base des prédictions fournies par le modèle mathématique MODEL ne permette pas de réguler correctement la glycémie du patient.

L'algorithme chapeau de minimisation d'hypoglycémie, également mis en oeuvre par le circuit de traitement et de contrôle 105, permet de prédire un risque imminent d'hypoglycémie, et, lorsqu'un tel risque est détecté, de sortir du mode de fonctionnement en commande prédictive basé sur l'utilisation du modèle mathématique MODEL, et de réduire ou interrompre le débit d'insuline injecté au patient, voire de proposer au patient un resucrage de façon à essayer d'éviter l'hypoglycémie.

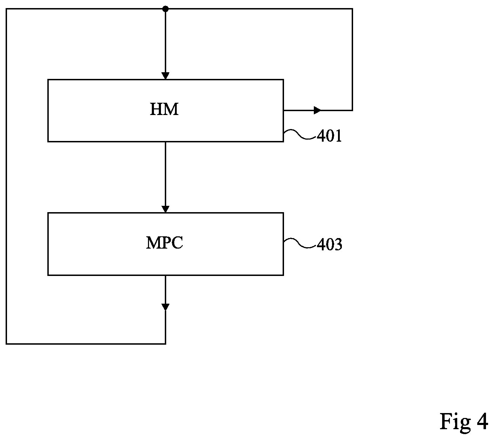

La figure 4 est un diagramme illustrant de façon simplifiée un exemple d'un mode de réalisation d'un procédé automatisé de régulation de glycémie mis en oeuvre par le système de la figure 1.

Le procédé de la figure 4 comprend une étape 401 (HM) correspondant à la mise en oeuvre de l'algorithme chapeau de minimisation d'hypoglycémie, et une étape 403 (MPC) correspondant à la mise en oeuvre d'un fonctionnement en commande prédictive basé sur l'utilisation du modèle mathématique MODEL tel que décrit ci-dessus en relation avec les figures 2 et 3.

Lors de l'étape 401, le circuit de traitement et de contrôle 105 détermine, sur la base des dernières données mémorisées pour le patient, s'il existe un risque d'hypoglycémie dans un avenir proche.

Si un tel risque est détecté, le circuit de traitement et de contrôle 105 réduit ou interrompt le débit d'insuline administré au patient au moyen du dispositif 103, et peut en outre, selon l'importance et/ou l'imminence de l'hypoglycémie prédite, proposer au patient un resucrage, par exemple au moyen d'un dispositif d'interface utilisateur (alarme, écran d'affichage, etc.) non détaillé ici. L'étape 401 est alors répétée jusqu'à ce que le risque d'hypoglycémie soit écarté.

Si, lors de l'étape 401, il n'est pas détecté de risque d'hypoglycémie dans un avenir proche, le dispositif de traitement et de contrôle 105 met en oeuvre l'étape 403. A titre d'exemple, l'étape 403 consiste en une unique itération des étapes 301, 303 et 305 du procédé de la figure 3. A l'issue de l'étape 403, l'étape 401 est à nouveau mise en oeuvre.

L'étape 401 (suivie le cas échéant de l'étape 403) est par exemple répétée à intervalles réguliers, par exemple à chaque nouvelle acquisition d'une valeur de glycémie par le capteur 101, par exemple toutes les 1 à 20 minutes, par exemple toutes les cinq minutes.

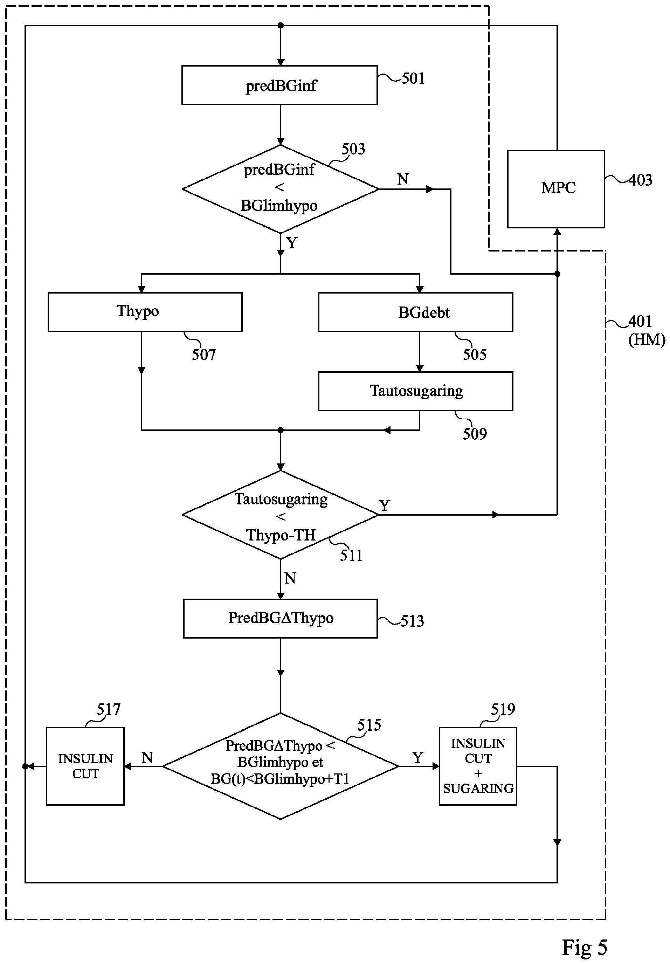

La figure 5 est un diagramme illustrant plus en détail un exemple de mise en oeuvre du procédé de régulation de la figure 4. Plus particulièrement, la figure 5 illustre de façon plus détaillée un exemple de réalisation de l'algorithme chapeau de minimisation d'hypoglycémie mis en oeuvre par le dispositif de traitement et de contrôle 105 lors de l'étape 401 du procédé de régulation de la figure 4.

Dans l'exemple de la figure 4, l'étape 401 comprend une première étape 501 de détermination, par l'unité de traitement et de contrôle 105, d'une valeur predBGinf correspondant à une prédiction de la glycémie du patient à l'horizon infini en l'absence de toute perturbation extérieure et notamment en l'absence de toute nouvelle ingestion de glucose et de toute nouvelle injection d'insuline, à l'exception d'un débit de base de référence du patient, qui correspond à un débit spécifique au patient, prescrit par son diabétologue et programmé pour être injecté par le dispositif 103 en l'absence de toute autre prise de contrôle par le système de régulation. Ainsi, la valeur predBGinf est représentative de la glycémie qu'aura le patient lorsque tout le glucose préalablement ingéré par le patient aura été assimilé et transformé en glucose sanguin.

La valeur predBGinf peut être déterminée en utilisant le modèle mathématique MODEL de la figure 2, ou tout autre modèle prédictif adapté.

Dans un mode de réalisation préféré, la valeur predBGinf est définie comme suit :

[Math 4]

$$predBGinf = BG(t0) - \frac{IOBimpact(t0)}{CR}$$

avec

[Math 5]

$$IOBimpact(t0) = IOB(t0) - COB(t0) * meal\_ratio$$

où :

- BG(t0) est la glycémie du patient à l'instant t0 d'observation, correspondant par exemple à la dernière valeur de glycémie mesurée par le capteur 101 ;
- IOB(t0) est une valeur représentative, à l'instant t0, de la quantité d'insuline embarquée ("insulin on board" en anglais) du patient, c'est-à-dire de la quantité d'insuline encore active (c'est-à-dire encore susceptible d'avoir un effet sur la glycémie) à l'instant t0 dans le corps du patient ;
- COB(t0) est une valeur représentative, à l'instant t0, de la quantité de glucose embarquée ("carbohydrate on board" en anglais) du patient, c'est-à-dire de la portion de la quantité de glucose ingérée par le patient encore susceptible, à l'instant t0, d'avoir un effet sur la glycémie du patient ;

- meal_ratio est un coefficient représentatif de la quantité de glucose ingéré compensée par une unité d'insuline (UI) pour le patient, par exemple en g/UI, UI désignant une unité internationale d'insuline, soit l'équivalent biologique d'environ 0,0347 mg d'insuline humaine ;
- CR désigne le coefficient de sensibilité à l'insuline du patient, représentatif de la quantité d'insuline nécessaire pour faire baisser la glycémie de un gramme par litre (en UI/g/l) ; et
- IOBimpact(t0) représente, à l'instant t0, la part de la quantité d'insuline embarquée IOB(t0) qui va conduire à diminuer la glycémie du patient par rapport à sa glycémie courante BG(t0).

Le signal IOB(t) est par exemple calculé par l'unité de traitement et de contrôle 105 à partir des données d'insuline injectée i(t) et d'une fonction d'action hIOB représentant l'évolution, en fonction du temps, de l'effet de l'insuline injectée sur l'absorption du glucose sanguin. A titre d'exemple, le signal IOB(t) est défini comme suit :

[Math 6]

$$IOB(t) = \sum_{k=0}^{K} i(t-k) * h_{IOB}(k)$$

où t est une variable temporelle discrétisée, K est un entier supérieur à 1 et k est un entier allant de 0 à K.

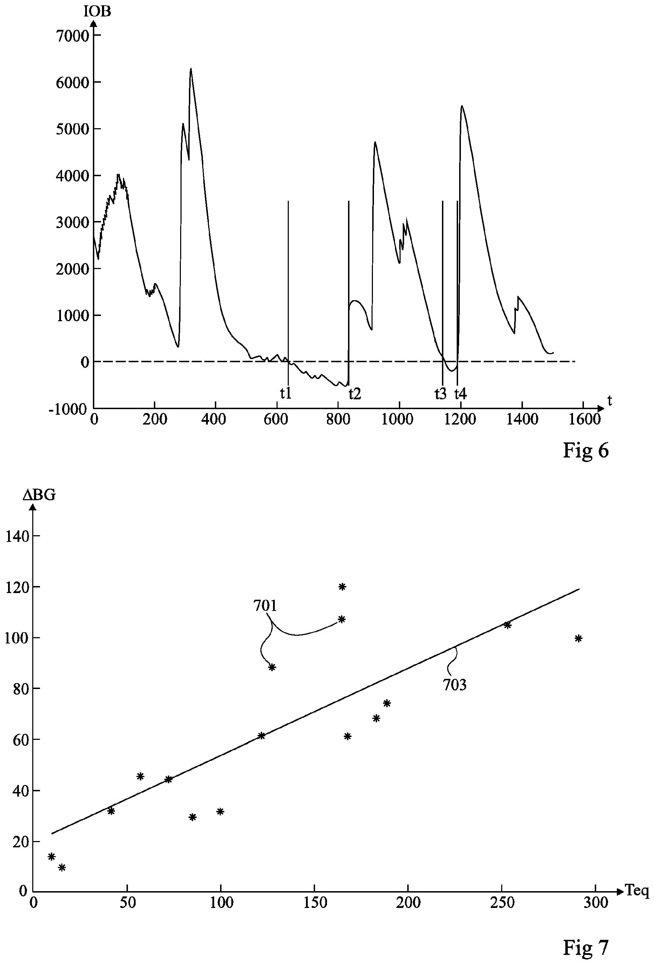

On notera que dans cet exemple, l'IOB est calculé hors débit de base de référence du patient. Autrement dit, pour le calcul de l'IOB, sur la base de l'équation Math 6 susmentionnée, la quantité d'insuline i(t) injectée considérée est en fait la différence entre la quantité totale iTOT(t) d'insuline injectée et le débit de base de référence iBASE(t) prescrit au patient. Cette quantité peut donc être négative lorsque le débit d'insuline du patient est interrompu. Ceci explique que l'IOB du patient puisse passer par des valeurs négatives comme cela sera décrit plus en détail ci-après en relation avec la figure 6.

Le signal COB(t) est par exemple calculé par l'unité de traitement et de contrôle 105 à partir des données de glucose ingéré cho(t) et d'une fonction d'action hCOB représentant l'évolution, en fonction du temps, de l'effet du glucose ingéré sur la glycémie. A titre d'exemple, le signal COB(t) est défini comme suit :

[Math 7]

$$COB(t) = \sum_{k=0}^{K} cho(t-k) * h_{COB}(k)$$

Dans les équations Math 6 et Math 7 susmentionnées, la grandeur K définit la durée d de prise en compte de l'historique d'insuline injectée ou de glucose ingéré pour le calcul des variables IOB(t) et COB(t), telle que d = K*T, T étant la période d'échantillonnage de la variable temps discrétisée. A titre d'exemple, la durée d est comprise entre 1 et 600 minutes, par exemple de l'ordre de 300 minutes.

Les fonctions d'action hIOB et hCOB sont par exemple définies comme suit :

[Math 8]

$$h_{IOB}(t) = \left[1 + \frac{t}{\tau IOB}\right] e^{-\frac{t}{\tau IOB}}$$

[Math 9]

$$h_{COB}(t) = \left[1 + \frac{t}{\tau COB}\right] e^{-\frac{t}{\tau COB}}$$

où $\tau IOB$ est une constante de temps, par exemple comprise entre 5 et 120 minutes, par exemple de l'ordre de 50 minutes, et où $\tau COB$ est une constante de temps, par exemple différente de $\tau IOB$, par exemple comprise entre 5 et 120 minutes, par exemple de l'ordre de 40 minutes.

Une fois la valeur predBGinf déterminée, le dispositif de traitement et de contrôle 105 met en oeuvre une étape 503 de comparaison de la valeur predBGinf à un seuil de glycémie BGlimhypo prédéterminé, correspondant au seuil en dessous duquel le patient est considéré comme étant en hypoglycémie, par exemple de l'ordre de 70 mg/dl.

Si, à l'étape 503, la valeur predBGinf est supérieure ou égale au seuil BGlimhypo (N), on considère qu'il n'y a pas de risque significatif d'hypoglycémie pour le patient dans un avenir proche. Dans ce cas, l'étape 401 se termine et le dispositif de traitement et de contrôle 105 met en oeuvre l'étape 403. Autrement dit, la régulation automatisée de la glycémie du patient est réalisée en utilisant le modèle mathématique MODEL, selon le mode de fonctionnement décrit en relation avec les figures 2 et 3.

Si, à l'étape 503, la valeur predBGinf est inférieure au seuil BGlimhypo (Y), on considère qu'il y a un risque que le patient tombe en hypoglycémie dans un avenir proche.

Le dispositif de traitement et de contrôle 105 met alors en oeuvre des étapes 505 et 507 visant à évaluer ce risque. Plus particulièrement, lors de l'étape 505, le dispositif 105 cherche à estimer quelle sera l'importance ou l'amplitude de l'hypoglycémie si rien n'est fait pour la stopper, et, lors de l'étape 507, le dispositif 105 cherche à déterminer dans combien de temps (à compter de l'instant t0), le patient entrera en hypoglycémie.

Lors de l'étape 505, le dispositif de traitement et de contrôle 105 détermine une valeur BGdebt correspondant à la dette de glycémie estimée du patient, c'est-à-dire l'écart entre la prévision de glycémie à l'horizon infini et le seuil d'hypoglycémie, définie comme suit :

[Math 10]

$$BGdebt = BGlimhypo - predBGinf$$

La valeur BGdebt correspond à un taux de glucose sanguin qu'il va falloir compenser pour éviter l'hypoglycémie.

Lors de l'étape 507, on cherche à estimer une durée Thypo restante (à compter de l'instant t0) avant le début de l'hypoglycémie.

Pour cela, le dispositif de traitement et de contrôle 105 détermine un coefficient négatif nGRC représentatif du taux de chute ou vitesse de diminution de la glycémie du patient, par exemple en mg/dl/min. Le temps Thypo restant avant l'hypoglycémie est alors calculé comme suit :

[Math 11]

$$\text{Thypo} = \frac{\text{BGlimhypo} - \text{BG(t0)}}{\text{nGRC}}$$

A titre d'exemple, le coefficient nGRC peut être choisi parmi les coefficients suivants :

- un coefficient nGRClim représentatif de la chute de glycémie maximale estimée pour le patient ;
- un coefficient nGRCmean représentatif de la chute de glycémie moyenne estimée pour le patient ; ou
- un coefficient nGRCcurrent représentatif de la chute courante de glycémie estimée pour le patient.

Les coefficients nGRClim et nGRCmean sont par exemple déterminés à partir d'un modèle spécifique au patient, construit sur la base d'un historique de données du patient, par exemple un historique de données enregistré par le système de régulation sur une période de plusieurs semaines à plusieurs mois. Au début de l'utilisation du système de régulation, avant qu'un historique de données suffisamment important ait été acquis, un modèle générique (i.e. non spécifique au patient), aussi appelé modèle population, peut être utilisé pour déterminer les coefficients nGRClim et nGRCmean. Le modèle population est par exemple déterminé à partir d'une base de données contenant l'historique de glycémie, l'historique d'injection d'insuline, et l'historique de prise de glucose d'un grand nombre de patients, par exemple au moins 20 patients, sur une période de relativement longue, par exemple de plusieurs semaines à plusieurs mois.

Le coefficient nGRCcurrent peut quant à lui être déterminé par extrapolation, par exemple linéaire, de la chute de glycémie mesurée par le capteur 101 au cours d'une période d'observation passée, par exemple de l'ordre de 10 à 40 minutes.

A titre de variante, au lieu de calculer une seule valeur estimée Thypo de durée restante avant l'hypoglycémie, sur la base de l'un des trois coefficients nGRClim, nGRCmean et nGRCcurrent susmentionnés, le circuit 105 peut être configuré pour calculer trois valeurs estimées de durée restante avant l'hypoglycémie Thypolim, Thypomean et Thypocurrent, sur la base respectivement des trois coefficients nGRClim, nGRCmean et nGRCcurrent.

Lors d'une étape 509 postérieure à l'étape 505, le dispositif de traitement et de contrôle 105 cherche à estimer en combien de temps la dette de glycémie BGdebt estimée lors de l'étape 505 pourrait être compensée par production endogène de glucose en cas de coupure du débit d'insuline injecté au patient. Il est en effet établi que la coupure du débit d'insuline permet de faire remonter la glycémie du fait que l'organisme, et notamment le foie, produit naturellement une quantité de glucose sanguin appelée EGP (de l'anglais "Endogeneous Glucose Production"). Ainsi, en l'absence d'injection d'insuline, un patient diabétique verra sa glycémie augmenter de façon sensiblement constante.

Lors de l'étape 509, le circuit 105 détermine une valeur Tautosugaring représentative de la durée nécessaire à la compensation naturelle de la dette de glycémie BGdebt par production endogène de glucose sanguin en cas de coupure du débit d'insuline. La durée Tautosugaring est calculée comme suit :

[Math 12]

$$\text{Tautosugaring} = \frac{\text{BGdebt}}{\text{EGE}}$$

où EGE (de l'anglais "Endogeneous Glucose Expansion") est une valeur représentative de la vitesse de remontée glycémique endogène du patient.

Des protocoles cliniques ont été proposés pour mesurer la vitesse de remontée glycémique endogène d'un patient. Ces protocoles sont toutefois contraignants et ne peuvent pas être répétés régulièrement.

Selon un aspect d'un mode de réalisation, on prévoit ici d'estimer la vitesse EGE du patient au moyen du dispositif de traitement et de contrôle 105 par analyse de l'historique de glycémie et de l'historique d'injection d'insuline du patient. Ceci permet d'estimer le paramètre EGE individuellement pour chaque patient, et de mettre à jour régulièrement ce paramètre au cas où sa valeur évoluerait dans le temps.

La méthode d'estimation du paramètre EGE mise en oeuvre par le dispositif 105 est illustrée par les figures 6 et 7.

La figure 6 est un diagramme illustrant l'évolution, en fonction du temps t (en abscisse, en minutes) de la quantité d'insuline embarquée IOB du patient (en ordonnée, en mUI). Comme indiqué précédemment, l'IOB considéré dans cet exemple est calculé hors débit de base de référence du patient (on parle aussi d'IOB net), ce qui explique que l'IOB du patient puisse passer par des valeurs négatives lorsque le débit d'insuline injecté au patient est interrompu ou réduit par rapport au débit de base de référence, comme cela apparaît sur la figure 6.

Pour estimer le paramètre EGE de vitesse de remontée glycémique endogène du patient, le dispositif 105 identifie des évènements de mesure, c'est-à-dire des plages de temps pendant lesquelles l'IOB du patient est négatif, c'est-à-dire des plages de temps pendant lesquelles le système de régulation a tenté de faire remonter naturellement la glycémie du patient par production endogène de glucose. Sur la figure 6, deux plages temporelles [t1, t2] et [t3, t4] pendant lesquelles la courbe IOB(t) est négative sont visibles (avec t1<t2<t3<t4).

Pour chaque évènement de mesure identifié, le dispositif 105 calcule une valeur Insulin_missed représentative de la quantité d'insuline manquante pour maintenir le niveau glycémique constant, définie comme suit :

[Math 13]

$$\text{Insulin\_missed} = \sum_{t=\text{tstart}}^{\text{tend}} \frac{d(\text{IOB}(t))}{dt}$$

où tstart et tend désignent respectivement l'instant de début et l'instant de fin de l'évènement de mesure (en considérant que l'activité d'insuline est la dérivée temporelle de l'IOB).

Une fois l'insuline manquante estimée, celle-ci peut être normalisée par rapport au débit de base de référence d'insuline du patient (en UI/h) pour se ramener à une grandeur temporelle équivalente Teq. La valeur Teq est alors représentative du temps équivalent de coupure du débit de base de référence du patient lié à l'évènement.

Une fois la valeur Teq déterminée, le circuit de traitement et de contrôle 105 mesure la remontée glycémique du patient liée à l'évènement, et en déduit l'augmentation endogène de glycémie du patient.

La figure 7 est un diagramme représentant, pour un patient donné, l'évolution, en fonction du temps équivalent de coupure du débit de base de référence Teq (en abscisse, en minutes), de la remontée naturelle de glycémie ΔG du patient pendant l'évènement (en ordonnée, en mg/dl). Le diagramme de la figure 7 comprend plus particulièrement un nuage de points 701, chaque point 701 correspondant à un évènement de mesure identifié par le dispositif 105 dans l'historique de données de glycémie et d'IOB du patient, et représentant le couple de valeur Teq, ΔG déterminé par le dispositif 105 pour cet évènement.

A partir de ce nuage de points, le dispositif 105 détermine, par régression linéaire, une droite 703 représentative de l'évolution de la remontée glycémique endogène ΔG du patient en fonction du temps équivalent Teq de coupure du débit de base de référence d'insuline.

La valeur affectée par l'unité de traitement et de contrôle 105 au paramètre EGE de vitesse de remontée glycémique endogène du patient correspond à la pente de la droite 703, de l'ordre de 27 mg/dl/h dans l'exemple de la figure 7.

La valeur du paramètre EGE peut par exemple être recalculée par le dispositif 105 à chaque nouvelle occurrence d'un évènement de mesure (i.e. passage de l'IOB du patient par une valeur négative).

A titre de variante, plutôt que de choisir la valeur du paramètre EGE comme étant égale à la pente de la courbe 703, le dispositif 105 peut affecter au paramètre EGE une valeur égale à la plus petite vitesse de remontée endogène de glycémie mesurée pour le patient lors des différents évènements de mesure identifiés.

Au début de l'utilisation du système, avant qu'un nombre significatif d'évènements de mesure ait pu être détecté par le dispositif 105, le dispositif 105 peut affecter au paramètre EGE une valeur fixe prédéterminée, par exemple une valeur déterminée selon le procédé ci-dessus mais en se basant sur l'historique d'IOB et l'historique de glycémie d'un grand nombre de patients.

Une fois la durée Tautosugaring (aussi appelée durée d'auto-resucrage) déterminée, le dispositif 105 met en oeuvre une étape 511 de comparaison de la durée Tautosugaring déterminée à l'étape 509 à la durée Thypo déterminée à l'étape 507. Plus particulièrement, dans cet exemple, le dispositif 105 compare la valeur Tautosugaring à une valeur Thypo - TH, où TH est une marge temporelle fixe prédéterminée, par exemple comprise entre 5 et 30 minutes, par exemple de l'ordre de 10 à 15 minutes.

Si, à l'étape 511, la valeur Tautosugaring est inférieure à la valeur Thypo - TH (Y), on considère qu'il n'y a pas de risque significatif d'hypoglycémie imminente pour le patient, et que l'hypoglycémie prédite à l'étape 503 peut encore être évitée en continuant à réguler la glycémie du patient sur la base du modèle MODEL. Autrement dit, l'hypoglycémie à venir détectée à l'étape 503 n'est pas considérée comme une menace à court terme dans la mesure où la dette glycémique du patient peut encore être compensée par interruption du débit d'insuline du patient. Dans ce cas, l'étape 401 se termine et le dispositif de traitement et de contrôle 105 met en oeuvre l'étape 403. Autrement dit, la régulation automatisée de la glycémie du patient est réalisée en utilisant le modèle mathématique MODEL, selon le mode de fonctionnement décrit en relation avec les figures 2 et 3.

Si, à l'étape 511, la valeur Tautosugaring est supérieure ou égale à la valeur Thypo - TH (N), on considère qu'il y a un risque que le patient entre en hypoglycémie dans un avenir proche.

Lors d'une étape 513, le dispositif 105 réalise alors une nouvelle prédiction de la glycémie future du patient, en considérant le débit d'injection d'insuline au patient entièrement interrompu. Plus particulièrement, le dispositif 105 calcule une valeur predBGΔThypo représentative de la glycémie future du patient à l'instant t0+Thypo.

La valeur predBGΔThypo peut être déterminée en utilisant le modèle MODEL de la figure 2, ou tout autre modèle prédictif adapté.

Dans un mode de réalisation préféré, la valeur predBGΔThypo est définie comme suit :

[Math 14]

$$predBG\Delta Thypo = BG(t0) - \frac{InsulinConsumed}{CR} + \frac{CHOconsumed}{SugaringRatio} + EGE * Thypo$$

où

[Math 15]

$$InsulinConsumed = \sum_{t=t0}^{t0+Thypo} \frac{d(IOB(t))}{dt}$$

et

[Math 16]

$$CHOconsumed = \sum_{t=t0}^{t0+Thypo} \frac{d(COB(t))}{dt}$$

et où SugaringRatio est un coefficient représentatif de l'impact du glucose ingéré sur la remontée glycémique, en g/g/l (gramme de CHO par g/l de glucose sanguin).

Cette prédiction permet, à chaque nouvelle itération de l'étape 401, de vérifier si l'hypoglycémie est en train d'être compensée.

Une fois la valeur predBGΔThypo déterminée, le dispositif de traitement et de contrôle 105 met en oeuvre une étape 515 de comparaison de la valeur predBGΔThypo avec le seuil d'hypoglycémie BGlimhypo.

A l'étape 515, le dispositif 105 détermine en outre si la glycémie courante BG(t0) du patient est suffisamment éloignée du seuil d'hypoglycémie BGlimhypo. Plus particulièrement, à l'étape 515, le dispositif 105 compare la valeur BG(t0) à une valeur BGlimhypo+T1, où T1 est une marge de glycémie prédéterminée, par exemple comprise entre 5 et 20 mg/dl.

Si, à l'étape 515, la valeur predBGΔThypo est supérieure ou égale au seuil BGlimhypo, ou si la valeur predBGΔThypo est inférieure au seuil BGlimhypo mais la valeur BG(t0) est supérieure ou égale à la valeur BGlimhypo+T1 (N), on considère que l'interruption du débit d'insuline peut encore suffire à éviter l'hypoglycémie.

Dans ce cas, lors d'une étape 517 (INSULIN CUT), le dispositif 105 commande l'interruption complète du débit d'insuline injecté au patient (y compris du débit de base de référence du patient). L'étape 401 est ensuite réitérée à partir de l'étape 501, par exemple après un intervalle compris entre 1 et 20 minutes, par exemple de l'ordre de 5 minutes, sans qu'un resucrage ne soit proposé au patient.

Si, à l'étape 515, la valeur predBGΔThypo est inférieure au seuil BGlimhypo et la valeur BG(t0) est inférieure à la valeur BGlimhypo+T1 (N), on considère que l'hypoglycémie est imminente et que la remontée glycémique naturelle par production endogène de glucose ne suffira pas à l'éviter.

Dans ce cas, lors d'une étape 519 (INSULIN CUT + SUGARING), le dispositif 105 commande alors l'interruption complète du débit d'insuline injecté au patient (y compris du débit de base de référence du patient), et commande en outre un dispositif d'interface utilisateur (non détaillé sur les figures), par exemple un écran d'affichage, une alarme, etc., pour indiquer au patient qu'il doit effectuer une prise de glucose pour éviter l'hypoglycémie. L'étape 401 est ensuite réitérée à partir de l'étape 501, par exemple après un intervalle compris entre 1 et 20 minutes, par exemple de l'ordre de 5 minutes.

Dans le cas où, à l'étape 507, le circuit 105 calcule trois valeurs estimées de durée restante avant l'hypoglycémie Thypolim, Thypomean et Thypocurrent, sur la base respectivement des trois coefficients nGRClim, nGRCmean et nGRCcurrent, on peut avantageusement prévoir :

- à l'étape 511, de comparer la durée Tautosugaring avec la valeur ThypoMIN-TH, où ThypoMIN est la plus courte des trois durées Thypolim, Thypomean et Thypocurrent ;
- à l'étape 513, de calculer trois valeurs estimées predBGΔThypolim, predBGΔThypomean et predBGΔThypocurrent de la glycémie future du patient respectivement aux instants t0+Thypolim, t0+Thypomean et t0+Thypocurrent ;
- à l'étape 515, de comparer chacune des trois valeurs predBGΔThypolim, predBGΔThypomean et predBGΔThypocurrent avec la valeur BGlimhypo et de passer à l'étape 517 (coupure d'insuline sans proposer de resucrage) uniquement si les trois valeurs predBGΔThypolim, predBGΔThypomean et predBGΔThypocurrent sont supérieures ou égales au seuil BGlimhypo ou si:

a) une seule des trois valeurs predBGΔThypolim, predBGΔThypomean et predBGΔThypocurrent est inférieure au seuil BGlimhypo et la valeur BG(t0) est supérieure ou égale à la valeur BGlimhypo+T1,
b) deux des trois valeurs predBGΔThypolim, predBGΔThypomean et predBGΔThypocurrent sont inférieures au seuil BGlimhypo et la valeur BG(t0) est supérieure ou égale à la valeur BGlimhypo+T2, où T2 est une marge de glycémie supérieure à la marge T1, ou
c) les trois valeurs predBGΔThypolim, predBGΔThypomean et predBGΔThypocurrent sont inférieures au seuil BGlimhypo et la valeur BG(t0) est supérieure ou égale à la valeur BGlimhypo+T3, où T3 est une marge de glycémie supérieure à la marge T2.

Dans le cas contraire (Y), un resucrage est proposé au patient en plus de la coupure d'insuline (étape 519).

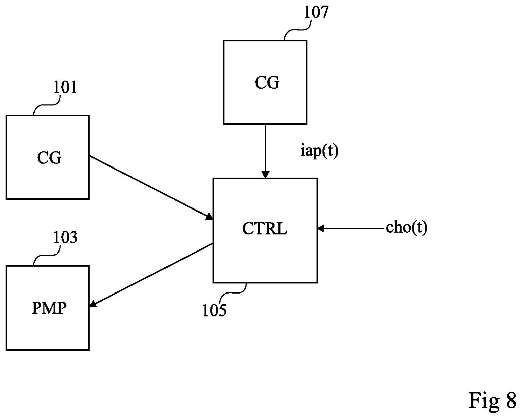

La figure 8 représente de façon schématique, sous forme de blocs, un autre exemple d'un système automatisé de régulation de la glycémie d'un patient selon un mode de réalisation. Le système de la figure 8 comprend les mêmes éléments que le système de la figure 1, agencés pour coopérer sensiblement de la même manière.

Dans l'exemple de la figure 8, le système comprend en outre un dispositif 107 de mesure d'une activité physique du patient. Le dispositif 107 est relié à l'unité de traitement et de contrôle 105, par exemple par liaison filaire ou par liaison radio (sans fil), et communique à l'unité de traitement et de contrôle 105 un signal iap(t) représentatif de l'évolution, en fonction du temps t, d'une activité physique du patient. Le dispositif 107 est par exemple un dispositif mobile porté par le patient tout au long de la journée et/ou de la nuit.

A titre d'exemple, le dispositif 107 est une simple interface utilisateur (non détaillée) par laquelle le patient déclare les activités physiques qu'il réalise. A titre d'exemple, le signal iap(t) correspond à un niveau d'intensité physique déclaré par le patient par le biais du dispositif 107. Le dispositif 107 est par exemple muni d'un clavier permettant à l'utilisateur de saisir, sur une échelle de 0 à N, où N est un entier strictement positif, par exemple égal à 3, le niveau d'intensité de l'activité physique qu'il est en train de réaliser, la valeur 0 correspondant à une activité physique considérée comme nulle ou négligeable, et la valeur N correspondant au niveau d'intensité d'activité physique maximal du patient.

A titre de variante, le dispositif 107 comprend un ou plusieurs capteurs adaptés à mesurer des grandeurs représentatives de l'activité physique du patient. A titre d'exemple, le dispositif 107 comprend au moins un capteur de mouvement (non détaillé sur la figure 8), par exemple un accéléromètre. Le dispositif 107 peut en outre comprendre un capteur du rythme cardiaque du patient (non détaillé sur la figure 8). Dans ce cas, le signal iap(t) est par exemple un signal représentatif de la dépense énergétique du patient, calculé à partir des données de sortie du ou des capteurs du dispositif 107, par exemple tel que décrit dans l'article intitulé "Prior automatic posture and activity identification improves physical activity energy expenditure prédiction from hip-worn triaxial accelerometry" de M. Garnotel et al. (Journal of Applied Physiology (1985). 2017 Nov 30), ou dans l'article intitulé "An original piecewise model for computing energy expenditure from accelerometer and heart rate signals" de H. Romero-Ugalde et al. (Physiological measurement, 2017 Jul 28;38(8):1599-1615).

A titre de variante, le signal iap(t) peut être une combinaison d'un signal mesuré au moyen d'un ou plusieurs capteurs du dispositif 107, et d'un signal d'intensité d'activité physique déclaré par le patient au moyen d'une interface utilisateur du dispositif 107.

Le fonctionnement du système de la figure 8 est similaire à ce qui a été décrit précédemment, à ceci près que, dans le système de la figure 8, le dispositif de traitement et de contrôle 105 tient compte du signal iap(t) lors de l'étape 401 du procédé de régulation de glycémie.

A titre d'exemple, à l'étape 501 de prédiction de la glycémie à l'horizon infini, le ou les coefficients nGRC, nGRClim, nGRCmean et nGRCcurrent représentatifs du taux de chute de la glycémie de patient sont adaptés en fonction du signal d'activité physique iap(t) du patient. En particulier, la ou les valeurs de taux de chute de glycémie utilisées seront choisies d'autant plus élevée que l'activité physique iap(t0) est élevée.

En outre ou de façon alternative, une nouvelle constante de temps d'action de l'insuline, diminuée pour modéliser l'augmentation de la vitesse d'action de l'insuline en présence d'une activité physique, peut être utilisée. A titre d'exemple, la constante de temps $\tau$IOB de l'équation Math 8 susmentionnée peut être remplacée par une constante de temps $\tau$IOBap = f(iap(t)), où f est une fonction décroissante modélisant l'augmentation de la vitesse d'action de l'insuline (et donc la diminution de la constante de temps d'action de l'insuline) lorsque l'intensité de l'activité physique iap(t) augmente.

A titre d'exemple, la fonction f est définie comme suit :

[Math 17]

$$\tau\text{IOBap} = f\big(\text{iap(t)}\big) = \frac{C}{\text{iap(t)}}$$

où C est un paramètre compris entre 1 et 100, par exemple sensiblement égal à 30.

On notera que le calcul ne se limite pas aux exemples de formules mathématiques [Math 6] et [Math 8] mentionnés ci-dessus pour définir le signal IOB(t). Plus généralement, ce mode de calcul pourra être utilisé quelle que soit la formule utilisée pour définir le signal IOB(t), comprenant la multiplication d'un signal représentatif de l'insuline injectée au patient par une exponentielle décroissance de constante $\tau$IOBap (de la forme e^(-t/$\tau$IOBap)).

Divers modes de réalisation et variantes ont été décrits. L'homme de l'art comprendra que certaines caractéristiques de ces divers modes de réalisation et variantes pourraient être combinées, et d'autres variantes apparaîtront à l'homme de l'art. En particulier, les modes de réalisation décrits ne se limitent pas aux exemples numériques mentionnés dans la présente description.

On notera en outre que dans les exemple décrits ci-dessus, le procédé automatisé de régulation de glycémie mis en oeuvre à l'étape 403, basé sur l'utilisation du modèle prédictif MODEL, peut être remplacé par tout autre procédé automatisé de régulation de glycémie, par exemple un procédé utilisant un algorithme de type matrice décisionnelle pour déterminer les doses d'insuline à administrer au patient, en fonction de divers paramètres observés tels que le niveau courant de glycémie mesuré par le capteur 101, ou encore la vitesse de variation (ou pente) de la glycémie sur une période passée.

De plus, dans le procédé de la figure 5, à l'étape 519, au lieu de proposer un resucrage au patient et d'interrompre le débit d'insuline injecté au patient, on pourra, à titre de variante, prévoir de proposer un resucrage au patient (via un dispositif d'interface utilisateur) sans couper le débit d'insuline injecté au patient. En effet, dans la pratique, l'effet du resucrage sur la glycémie du patient est bien plus rapide que la remontée glycémique endogène du patient, de sorte que, en cas de resucrage, l'hypoglycémie peut être évitée sans interrompre le débit d'insuline du patient.

**Revendications**

1. Système automatisé de régulation de la glycémie d'un patient, comportant :

   - un capteur de glycémie (101) ;
   - un dispositif d'injection d'insuline (103) ; et
   - une unité de traitement et de contrôle (105),

   dans lequel l'unité de traitement et de contrôle (105) est configurée pour mettre en oeuvre un procédé de minimisation d'hypoglycémie (HM) comprenant les étapes suivantes :

   a) détecter (501, 503) une possible hypoglycémie à venir à partir de mesures de glycémie fournies par le capteur de glycémie ;
   b) déterminer (505, 507) une valeur BGdebt représentative d'une dette de glycémie à compenser pour éviter l'hypoglycémie détectée à l'étape a) et une valeur Thypo représentative de la durée restante avant le début de l'hypoglycémie détectée à l'étape a) ;
   c) déterminer (509) une valeur Tautosugaring représentative de la durée nécessaire pour permettre au patient de compenser la dette de glycémie BGdebt déterminée à l'étape b) par production endogène de glucose ;
   d) comparer (511) la valeur Tautosugaring déterminée à l'étape c) à une valeur Thypo-TH, où TH est une marge temporelle prédéterminée ; et
   e) lorsqu'il est déterminé à l'étape d) que la valeur Tautosugaring est supérieure ou égale à la valeur Thypo-TH, commander (517, 519) l'interruption du débit d'insuline injecté au patient par le dispositif d'injection d'insuline (103) et/ou déclencher une alerte via un dispositif d'interface utilisateur du système pour indiquer au patient qu'il doit procéder à un resucrage, dans lequel, à l'étape b), la valeur Thypo est calculée comme suit :

[Math 11]

$$\text{Thypo} = \frac{\text{BGlimhypo} - \text{BG(t0)}}{\text{nGRC}}$$

où BGlimhypo est un seuil d'hypoglycémie prédéterminé, BG(t0) est une valeur représentative de la glycémie courante du patient, mesurée par le capteur, et nGRC est un coefficient négatif représentatif du taux de chute de la glycémie du patient,
et dans lequel, à l'étape c) la valeur Tautosugaring est calculée comme suit :

[Math 12]

$$\text{Tautosugaring} = \frac{\text{BGdebt}}{\text{EGE}}$$

où EGE est une valeur représentative de la vitesse de remontée glycémique endogène du patient.

2. Système selon la revendication 1, dans lequel, à l'étape c), l'unité de traitement et de contrôle (105) détermine la valeur EGE représentative de la vitesse de remontée glycémique endogène du patient.

3. Système selon la revendication 2, dans lequel, à l'étape c), la valeur EGE est calculée par l'unité de traitement et de contrôle (105) par analyse de l'historique de glycémie et de l'historique d'injection d'insuline du patient.

4. Système selon l'une quelconque des revendications 1 à 3, dans lequel, à l'étape e), l'unité de traitement et de contrôle (105) détermine une valeur predBG∆Thypo estimative de la glycémie future du patient à l'issue de la période Thypo.

5. Système selon la revendication 4, dans lequel l'unité de traitement et de contrôle (105) est configurée pour, après l'étape e), mettre en oeuvre les étapes suivantes :

   f) comparer (515) la valeur predBG∆Thypo au seuil d'hypoglycémie BGlimhypo prédéterminé ;
   g) lorsqu'il est déterminé à l'étape f) que la valeur predBG∆Thypo est inférieure au seuil d'hypoglycémie BGlimhypo, comparer une valeur BG(t0) représentative de la glycémie courante du patient, mesurée par le capteur (101), à une valeur BGlimhypo+T1, où T1 est une marge de glycémie prédéterminée ; et
   h) lorsqu'il est déterminé à l'étape g) que la valeur BG(t0) est inférieure à la valeur BGlimhypo+T1, déclencher (519) une alerte via un dispositif d'interface utilisateur du système pour indiquer au patient qu'il doit procéder à un resucrage.

6. Système selon l'une quelconque des revendications 1 à 5, dans lequel, à l'étape b), l'unité de traitement et de contrôle (105) détermine trois valeurs Thypolim, Thypomean et Thypocurrent estimatives de la durée restante avant le début de l'hypoglycémie, basées respectivement sur un taux de chute de glycémie maximal nGRClim déterminé pour le patient, sur un taux de chute de glycémie moyen nGRCmean estimé pour le patient, et sur un taux de chute de glycémie courant nGRCcurrent estimé pour le patient.

7. Système selon la revendication 6, dans lequel l'unité de traitement et de contrôle (105) est configurée pour, à l'étape d), comparer la durée Tautosugaring à une valeur ThypoMIN-TH, où ThypoMIN est la plus courte des trois durées Thypolim, Thypomean et Thypocurrent.

8. Système selon la revendication 7, dans lequel l'unité de traitement et de contrôle (105) est configurée pour, à l'étape e), calculer trois valeurs predBG∆Thypolim, predBG∆Thypomean et predBG∆Thypocurrent estimatives de la glycémie future du patient respectivement à l'issue de la période Thypolim, à l'issue de la période Thypomean et à l'issue de la période Thypocurrent.

9. Système selon la revendication 8, dans lequel l'unité de traitement et de contrôle est configurée pour, après l'étape e), mettre en oeuvre les étapes suivantes :

f') comparer chacune des trois valeurs predBGΔThypolim, predBGΔThypomean et predBGΔThypocurrent au seuil d'hypoglycémie BGlimhypo prédéterminé ; et

g') si une seule des trois valeurs predBGΔThypolim, predBGΔThypomean et predBGΔThypocurrent est inférieure au seuil BGlimhypo, proposer un resucrage au patient uniquement si une valeur BG(t0) représentative de la glycémie courante du patient est inférieure à une valeur BGlimhypo+T1, où T1 est une marge de glycémie prédéterminée, si deux des trois valeurs predBGΔThypolim, predBGΔThypomean et predBGΔThypocurrent sont inférieures au seuil BGlimhypo, proposer un resucrage au patient (519) uniquement si la valeur BG(t0) est inférieure à une valeur BGlimhypo+T2, où T2 est une marge de glycémie prédéterminée supérieure à la marge T1, et si les trois valeurs predBGΔThypolim, predBGΔThypomean et predBGΔThypocurrent sont inférieures au seuil BGlimhypo, proposer un resucrage au patient uniquement si la valeur BG(t0) est inférieure à une valeur BGlimhypo+T3, où T3 est une marge de glycémie prédéterminée supérieure à la marge T2.

10. Système selon l'une quelconque des revendications 1 à 9, comprenant en outre un dispositif (107) de mesure d'une activité physique du patient relié à l'unité de traitement et de contrôle (105) et communiquant à l'unité de traitement et de contrôle (105) un signal iap(t) représentatif de l'évolution, en fonction du temps, d'une activité physique du patient.

11. Système selon la revendication 10, dans lequel l'unité de traitement et de contrôle (105) tient compte du signal iap(t) pour détecter une possible hypoglycémie à venir à l'étape a) et/ou pour déterminer la valeur Tautosugaring à l'étape c).

12. Système selon la revendication 11, dans lequel, à l'étape c), l'unité de traitement et de contrôle (105) détermine un signal IOB(t) représentatif de la quantité d'insuline embarquée du patient, défini par multiplication d'un signal représentatif de la quantité d'insuline injectée au patient par une exponentielle décroissante de constante de temps τIOBap, la constante de temps τIOBap étant une fonction décroissante du signal iap(t), modélisant l'augmentation de la vitesse d'action de l'insuline lorsque l'intensité de l'activité physique augmente.

13. Système selon la revendication 12, dans lequel la constante de temps τIOBap est définie comme suit :

[Math 19]

$$\tau IOBap = \frac{C}{iap(t)}$$

où C est un paramètre compris entre 1 et 100.

**Patentansprüche**

1. Automatisiertes System zur Regulierung des Blutglukosespiegels eines Patienten, das Folgendes aufweist:

- einen Blutglukosesensor (101);
- eine Insulininjektionsvorrichtung (103); und
- eine Verarbeitungs- und Steuereinheit (105),

wobei die Verarbeitungs- und Steuereinheit (105) konfiguriert ist, ein Hypoglykämie-Minimierungsverfahren (HM) zu implementieren, das die folgenden Schritte aufweist:

a) Erfassen (501, 503) einer möglicherweise eintretenden Hypoglykämie auf Grundlage von Blutglukosemessungen, die von dem Blutglukosesensor geliefert werden;
b) Bestimmen (505, 507) eines Wertes BGdebt, der eine zu kompensierende Blutglukoseschuld darstellt, um die in Schritt a) erfasste Hypoglykämie zu vermeiden, und eines Wertes Thypo, der die verbleibende Zeit vor dem Beginn der in Schritt a) erfassten Hypoglykämie darstellt;

c) Bestimmen (509) eines Wertes Tautosugaring, der für die Dauer repräsentativ ist, die erforderlich ist, um den Patienten in die Lage zu versetzen, die in Schritt b) bestimmte Blutglukoseschuld BGdebt durch endogene Glukoseproduktion auszugleichen;

d) Vergleichen (511) des in Schritt c) bestimmten Wertes Tautosugaring mit einem Wert Thypo-TH, wobei TH eine vorbestimmte Zeitspanne ist; und

e) wenn in Schritt d) festgestellt wird, dass der Wert Tautosugaring größer oder gleich dem Wert Thypo-TH ist, Steuern (517, 519) der Unterbrechung des Insulinflusses, der dem Patienten durch die Insulininjektionsvorrichtung (103) injiziert wird, und/oder Starten eines Alarms über eine Benutzerschnittstellenvorrichtung des Systems, um den Patienten darauf hinzuweisen, dass er ein Resugaring durchführen muss, wobei in Schritt b) der Wert Thypo wie folgt berechnet wird:

[Math 11]

$$Thypo = \frac{BGlimhypo - BG(t0)}{nGRC}$$

wobei BGlimhypo ein vorbestimmter Hypoglykämie-Schwellenwert ist, wobei BG(to) ein Wert ist, der für die aktuelle Blutglukose des Patienten repräsentativ ist, der von dem Sensor gemessen wird, wobei nGRC ein negativer Koeffizient ist, der für den Blutglukoseabfall des Patienten repräsentativ ist, und wobei in Schritt c) der Wert Tautosugaring wie folgt berechnet wird:

[Math 18]

$$Tautosugaring = \frac{BGdebt}{EGE}$$

wobei EGE ein Wert ist, der für die endogene Blutglukoseanstiegsrate des Patienten repräsentativ ist.

2. System nach Anspruch 1, wobei in Schritt c) die Verarbeitungs- und Steuereinheit (105) den Wert EGE bestimmt, der für die endogene Blutglukoseanstiegsrate des Patienten repräsentativ ist.

3. System nach Anspruch 2, wobei in Schritt c) der Wert EGE von der Verarbeitungs- und Steuereinheit (105) durch Analyse der Blutglukosehistorie des Patienten und der Insulininjektionshistorie berechnet wird.

4. System nach einem der Ansprüche 1 bis 3, wobei in Schritt e) die Verarbeitungs- und Steuereinheit (105) einen Wert predBG∆Thypo bestimmt, der die zukünftige Blutglukose des Patienten am Ende einer Periode Thypo schätzt.

5. System nach Anspruch 4, wobei die Verarbeitungs- und Steuereinheit (105) so konfiguriert ist, dass sie nach Schritt e) die folgenden Schritte ausführt:

f) Vergleichen (515) des Wertes predBG∆Thypo mit dem vorbestimmten Hypoglykämie-Schwellenwert BGlimhypo;

g) wenn in Schritt f) festgestellt wird, dass der Wert predBG∆Thypo kleiner ist als der Hypoglykämie-Schwellenwert BGlimhypo, Vergleichen eines Wertes BG(t0), der für die aktuelle Blutglukose des Patienten repräsentativ ist, und zwar gemessen von dem Sensor (101), mit einem Wert BGlimhypo+ T1, wobei T1 eine vorbestimmte Blutglukosespanne ist; und

h) wenn in Schritt g) festgestellt wird, dass der Wert BG(t0) kleiner ist als der Wert BGlimhypo+T1, Starten (519) eines Alarms über eine Benutzerschnittstellenvorrichtung des Systems, um den Patienten darauf hinzuweisen, dass er ein Resugaring durchführen muss.

6. System nach einem der Ansprüche 1 bis 15, wobei in Schritt b) die Verarbeitungs- und Steuereinheit (105) drei Werte Thypolim, Thypomean und Thypocurrent bestimmt, die die verbleibende Zeit bis zum Beginn der Hypoglykämie abschätzen, und zwar auf Grundlage einer für den Patienten bestimmten maximalen Blutglukoseabfallrate nGRClim, einer für den Patienten geschätzten durchschnittlichen Blutglukoseabfallrate nGRCmean und einer für den Patienten geschätzten aktuellen Blutglukoseabfallrate nGRCcurrent.

**7.** System nach Anspruch 6, wobei die Verarbeitungs- und Steuereinheit (105) so konfiguriert ist, dass sie in Schritt d) die Dauer Tautosugaring mit einem Wert ThypoMIN-TH vergleicht, wobei ThypoMIN die kürzeste der drei Dauern Thypolim, Thypomean und Thypocurrent ist.

**8.** System nach Anspruch 7, wobei die Verarbeitungs- und Steuereinheit (105) so konfiguriert ist, dass sie in Schritt e) drei Werte predBGΔThypolim, predBGΔThypomean und predBGΔThypocurrent berechnet, die den zukünftigen Blutglukose des Patienten jeweils am Ende der Periode Thypolim, am Ende der Periode Thypomean und am Ende der Periode Thypocurrent schätzen.

**9.** System nach Anspruch 8, wobei die Verarbeitungs- und Steuereinheit so konfiguriert ist, dass sie nach Schritt e) die folgenden Schritte ausführt:

f') Vergleichen von jedem der drei Werte predBGΔThypolim, predBGΔThypomean und predBGΔThypocurrent mit dem vorbestimmten Hypoglykämie-Schwellenwert BGlimhypo; und

g') wenn ein einziger der drei Werte predBGΔThypolim, predBGΔThypomean und predBGΔThypocurrent kleiner als der Schwellenwert BGlimhypo ist, nur dann dem Patienten ein Resugaring Vorschlagen, wenn ein Wert BG(t0), der die aktuelle Blutglukose des Patienten repräsentiert, kleiner als ein Wert BGlimhypo+T1 ist, wobei T1 eine vorbestimmte Blutglukosespanne ist,

wenn zwei der drei Werte predBGΔThypolim, predBGΔThypomean und predBGΔThypocurrent kleiner als der Schwellenwert BGlimhypo sind, nur dann dem Patienten ein Resugaring Vorschlagen (519), wenn der Wert BG(t0) kleiner ist als ein Wert BGlimhypo+T2, wobei T2 eine bestimmte Blutglukosespanne ist, die größer ist als die Spanne T1, und

wenn die drei Werte predBGΔThypolim, predBGΔThypomean, und predBGΔThypocurrent kleiner sind als der Schwellenwert BGlimhypo, nur dann dem Patienten ein Resugaring Vorschlagen, wenn der Wert BG(t0) kleiner ist als ein Wert BGlimhypo+T3, wobei T3 eine vorbestimmte Blutglukosespanne größer als die Spanne T2 ist.

**10.** System nach einem der Ansprüche 1 bis 9, das ferner eine Vorrichtung (107) zum Messen einer körperlichen Aktivität des Patienten aufweist, die mit der Verarbeitungs- und Steuereinheit (105) gekoppelt ist und die der Verarbeitungs- und Steuereinheit (105) ein Signal iap(t) übermittelt, das die zeitliche Veränderung einer körperlichen Aktivität des Patienten darstellt.

**11.** System nach Anspruch 10, wobei die Verarbeitungs- und Steuereinheit (105) das Signal iap(t) berücksichtigt, um eine möglicherweise eintretende Hypoglykämie in Schritt a) zu erkennen und/oder um den Wert Tautosugaring in Schritt c) zu bestimmen.

**12.** System nach Anspruch 11, wobei die Verarbeitungs- und Steuereinheit (105) im Schritt c) ein Signal IOB(t) bestimmt, das für eine On-Bord-Insulinmenge des Patienten repräsentativ ist, die definiert ist durch die Multiplikation eines Signals, das für die dem Patienten injizierte Insulinmenge repräsentativ ist, mittels einer abnehmenden Exponentialfunktion einer Zeitkonstante τIOBap definiert ist, wobei die Zeitkonstante τIOBap eine abnehmende Funktion des Signals iap(t) ist, die die Zunahme der Wirkungsgeschwindigkeit von Insulin modelliert, wenn die Intensität der körperlichen Aktivität zunimmt.

**13.** System nach Anspruch 12, wobei die Zeitkonstante τIOBap wie folgt definiert ist:

[Math 19]

$$\tau IOBap = \frac{C}{iap(t)}$$

wobei C ein Parameter im Bereich von 1 bis 100 ist.

**Claims**

**1.** Automated system of regulation of a patient's blood glucose, comprising:

- a blood glucose sensor (101);
- an insulin injection device (103); and
- a processing and control unit (105),

wherein the processing and control unit (105) is configured to implement a hypoglycemia minimization method (HM) comprising the steps of:

a) detecting (501, 503) a possible hypoglycemia to come based on blood glucose measurements supplied by the blood glucose sensor;
b) determining (505, 507) a value BGdebt representative of a blood glucose debt to be compensated to avoid the hypoglycemia detected at step a) and a value Thypo representative of the time remaining before the beginning of the hypoglycemia detected at step a);
c) determining (509) a value Tautosugaring representative of the duration necessary to enable the patient to compensate for the blood glucose debt BGdebt determined at step b) by endogenous glucose production;
d) comparing (511) the value Tautosugaring determined at step c) with a value Thypo-TH, where TH is a predetermined time margin; and
e) when it is determined at step d) that value Tautosugaring is greater than or equal to value Thypo-TH, controlling (517, 519) the interruption of the insulin flow injected to the patient by the insulin injection device (103) and/or starting an alert via a user interface device of the system to indicate to the patient that they must perform a resugaring, wherein, at step b), the value Thypo is calculated as follows:

[Math 11]

$$\text{Thypo} = \frac{\text{BGlimhypo} - \text{BG(t0)}}{\text{nGRC}}$$

where BGlimhypo is a predetermined hypoglycemia threshold, BG(to) is a value representative of the the patient's current blood glucose, measured by the sensor, nGRC is a negative coefficient representative of the patient's blood glucose drop,
and wherein, at step c), value Tautosugaring is calculated as follows:

[Math 18]

$$\text{Tautosugaring} = \frac{\text{BGdebt}}{\text{EGE}}$$

where EGE is a value representative of the patient's endogenous blood glucose rise rate.

2. System according to claim 1, wherein, at step c), the processing and control unit (105) determines the value EGE representative of the patient's endogenous blood glucose rise rate.

3. System according to claim 2, wherein, at step c), value EGE is calculated by the processing and control unit (105) by analysis of the patient's blood glucose history and insulin injection history.

4. System according to any of claims 1 to 3, wherein, at step e), the processing and control unit (105) determines a value predBGΔThypo estimative of the patient's future blood glucose at the end of period Thypo.

5. System according to claim 4, wherein the processing and control unit (105) is configured to, after step e), implement the steps of:

f) comparing (515) value predBGΔThypo with the predetermined hypoglycemia threshold BGlimhypo;
g) when it is determined at step f) that value predBGΔThypo is smaller than hypoglycemia threshold BGlimhypo, comparing a value BG(t0) representative of the patient's current blood glucose, measured by the sensor (101), with a value BGlimhypo+T1, where T1 is a predetermined blood glucose margin; and
h) when it is determined at step g) that value BG(t0) is smaller than value BGlimhypo+T1, start (519) an alert via a user interface device of the system to indicate to the patient that they must perform a resugaring.

6. System according to any of claims 1 to 15, wherein, at step b), the processing and control unit (105) determines three values Thypolim, Thypomean, and Thypocurrent estimative of the time remaining before the beginning of the hypoglycemia, respectively based on a maximum blood glucose drop rate nGRClim determined for the patient, on an average blood glucose drop rate nGRCmean estimated for the patient, and on a current blood glucose drop rate nGRCcurrent estimated for the patient.

7. System according to claim 6, wherein the processing and control unit (105) is configured to, at step d), compare duration Tautosugaring with a value ThypoMIN-TH, where ThypoMIN is the shortest of the three durations Thypolim, Thypomean, and Thypocurrent.

8. System according to claim 7, wherein the processing and control unit (105) is configured to, at step e), calculate three values predBGΔThypolim, predBGΔThypomean, and predBGΔThypocurrent estimative of the patient's future blood glucose respectively at the end of period Thypolim, at the end of period Thypomean, and at the end of period Thypocurrent.

9. System according to claim 8, wherein the processing and control unit is configured to, after step e), implement the steps of:

   f') comparing each of the three values predBGΔThypolim, predBGΔThypomean, and predBGΔThypocurrent with the predetermined hypoglycemia threshold BGlimhypo; and
   g') if a single one of the three values predBGΔThypolim, predBGΔThypomean, and predBGΔThypocurrent is smaller than threshold BGlimhypo, suggesting a resugaring to the patient only if a value BG(t0) representative of the patient's current blood sugar is smaller than a value BGlimhypo+T1, where T1 is a predetermined blood sugar margin, if two of the three values predBGΔThypolim, predBGΔThypomean, and predBGΔThypocurrent are smaller than threshold BGlimhypo, suggesting a resugaring to the patient (519) only if value BG(t0) is smaller than a value BGlimhypo+T2, where T2 is a determined blood glucose margin greater than margin T1, and if the three values predBGΔThypolim, predBGΔThypomean, and predBGΔThypocurrent are smaller than threshold BGlimhypo, suggesting a resugaring to the patient only if value BG(t0) is smaller than a value BGlimhypo+T3, where T3 is a predetermined blood glucose margin greater than margin T2.

10. System according to any of claims 1 to 9, further comprising a device (107) for measuring a physical activity of the patient coupled to the processing and control unit (105) and communicating to the processing and control unit (105) a signal iap(t) representative of the time variation of a physical activity of the patient.

11. System according to claim 10, wherein the processing and control unit (105) takes into account signal iap(t) to detect a possible hypoglycemia to come at step a) and/or to determine value Tautosugaring at step c).

12. System according to claim 11, wherein, at step c), the processing and control unit (105) determines a signal IOB(t) representative of the patient's quantity of insulin on board, defined by the multiplication of a signal representative of the quantity of insulin injected to the patient by a decreasing exponential of time constant τIOBap, time constant τIOBap being a decreasing function of signal iap(t), modeling the increase of the speed of action of insulin when the intensity of the physical activity increases.

13. System according to claim 12, wherein time constant τIOBap is defined as follows:

[Math 19]

$$\tau IOBap = \frac{C}{iap(t)}$$

where C is a parameter in the range from 1 to 100.

Fig 1

Fig 2

Fig 3

Fig 4

Fig 5

Fig 6

Fig 7

Fig 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2018055283 A
- DD 16959B15018
- WO 2018055284 A
- DD 17175B15267
- US 2014118138 A1, COBELLI

**Littérature non-brevet citée dans la description**

- **ROMAN HOVORKA et al.** Nonlinear model prédictive control of glucose concentration in subjects with type 1 diabètes. *Physiol Meas,* 2004, vol. 25, 905-920
- **ROMAN HOVORKA et al.** Partitioning glucose distribution/transport, disposal, and endogenous production during IVGTT. *Am J Physiol Endocrinol Metab,* 2002, vol. 282, E992-E1007
- **CHIARA DALLA MAN et al.** A System Model of Oral Glucose Absorption: Validation on Gold Standard Data. *IEEE TRANSACTIONS ON BIOMEDICAL ENGINEERING,* Décembre 2006, vol. 53 (12
- **M. GARNOTEL et al.** Prior automatic posture and activity identification improves physical activity energy expenditure prédiction from hip-worn triaxial accelerometry. *Journal of Applied Physiology,* 1985
- **H. ROMERO-UGALDE et al.** An original piecewise model for computing energy expenditure from accelerometer and heart rate signals. *Physiological measurement,* 28 Juillet 2017, vol. 38 (8), 1599-1615